# EUROPEAN PATENT APPLICATION

(11) **EP 2 957 211 A1**
(43) Date of publication of application: **23.12.2015**
(21) Application number: 14751587.8
(22) Date of filing: 06.02.2014
(51) Int. Cl.: A61B 1/00, A61B 1/04

(54) **FLUORESCENT LIGHT OBSERVATION DEVICE**

(30) Priority: 13.02.2013 JP 2013025387
(71) Applicant: Olympus Corporation, Shibuya-ku Tokyo 151-0072 (JP)
(72) Inventor: MORISHITA, Koki, Tokyo 151-0072 (JP)
(74) Representative: Schicker, Silvia
(86) International application number: PCT/JP2014/052808
(87) International publication number: WO 2014/126000

(57) **Abstract**

A fluoroscopy apparatus (1) of the present invention is provided with a light-source portion (3) that irradiates biological tissue (X) with illumination light (L) including excitation light, a return-light-image generating portion (61) that generates a return-light image (G1) by capturing return light of the illumination light (L) from the biological tissue (X), and a fluorescence-image generating portion (62) that generates a fluorescence image (G2) by capturing fluorescence from the biological tissue (X), wherein the illumination light (L) is visible light that does not include at least a portion of wavelength region between 490 nm and 540 nm, and the excitation light includes some wavelengths of the illumination light (L) and generates fluorescence having a wavelength included in the at least a portion of the wavelength region.

## Description

### {Technical Field}

The present invention relates to a fluoroscopy apparatus.

### {Background Art}

In the related art, there are known fluoroscopy apparatuses that alternately acquire both white-light images and fluorescence images of biological tissue in a time-division manner (for example, see Patent Literature 1). According to Patent Literature 1, it is possible to separately capture fluorescence and white light, even in the case in which the wavelength of the fluorescence is in the visible region and the wavelength of the fluorescence overlaps with some wavelengths of the white light.

### {Citation List}

### {Patent Literature}

{PTL 1} Publication of Japanese Patent No. 4520216

### {Summary of Invention}

### {Technical Problem}

However, with Patent Literature 1, because the white-light images and the fluorescence images are alternately acquired, there is a problem in that the frame rates are decreased for the individual images. In particular, because an observer performs observation by mainly using the white-light images, the decreased frame rates of the white-light images act as a hindrance for the observer when performing observation.

The present invention has been conceived in light of the above-described circumstances, and an object thereof is to provide a fluoroscopy apparatus with which it is possible to concurrently acquire a white-light image and a visible-region fluorescence image without decreasing the frame rate of the white-light image.

### {Solution to Problem}

In order to achieve the above-described object, the present invention provides the following solutions.

The present invention provides a fluoroscopy apparatus including a return-light-image generating portion that generates a return-light image of the biological tissue by capturing return light returning from the biological tissue irradiated with the illumination light from the light-source portion; and a fluorescence-image generating portion that generates a fluorescence image by capturing fluorescence generated at the biological tissue due to irradiation thereof with the excitation light from the light-source portion, wherein the illumination light is visible light that does not include at least a portion of the wavelength region between 490 nm and 540 nm, and the excitation light includes some wavelengths of the illumination light and generates fluorescence having a wavelength included in the at least a portion of the wavelength region.

With the present invention, by irradiating the biological tissue with the illumination light from the light-source portion, the return-light-image generating portion generates a return-light image in which the morphology of the biological tissue is captured. Simultaneously with this, by irradiating the biological tissue with the excitation light included in the illumination light, the fluorescence-image generating portion acquires a fluorescence image in which the fluorescence from the biological tissue is captured.

In this case, because the return light and the fluorescence both have wavelengths in the visible region, but have wavelengths differing from each other, the return-light-image generating portion and the fluorescence-image generating portion capture the return light and the fluorescence by distinguishing between the two. Accordingly, it is possible to concurrently acquire the return-light image and the fluorescence image without decreasing the frame rates of the return-light image. In addition, the light of the wavelengths between 490 nm and 530 nm has a sufficiently low effect on the biological tissue, and thus, this light makes almost no contribution to the acquisition of the morphological information of the biological tissue. Therefore, even if the illumination light does not include some wavelengths between 490 nm and 540 nm, it is possible to acquire a return-light image that is equivalent to a white-light image in which the morphology of the biological tissue is sufficiently clearly captured.

In the above-described invention, the at least a portion of the wavelength region may be 510 nm to 530 nm.

In the above-described invention, the at least a portion of the wavelength region may have a width equal to or greater than 20 nm.

In the above-described invention, the light-source portion may be provided with a white-light source that emits white light and a filter that removes light of the at least a portion of the wavelength region between 490 nm and 540 nm from the white light emitted from the white-light source, wherein the filter may be provided at a post-stage of the white-light source in an insertable/removable manner, and the illumination light may be substantially white light that has passed through the filter.

By doing so, when not acquiring a fluorescence image, the biological tissue is irradiated with the white light that includes the wavelength covering the entire visible region as the illumination light by removing the filter from the post-stage of the white-light source, and thus, it is possible to acquire a return-light image in which the color of the biological tissue is more accurately produced. On the other hand, when acquiring a fluorescence image, the biological subject is irradiated with the substantially white light, which is the white light from which the portion of the wavelength region is removed, as the illumination light by inserting the filter at the post-stage of the white-light source, and thus, it is possible to acquire a return-light image in which the color of the biological tissue is substantially accurately produced.

The above-described invention may be provided with a white-balance switching portion that switches white balances of the return-light images generated by the return-light-image generating portion, when the filter is inserted at the post-stage of the white-light source and when the filter is removed from the post-stage of the white-light source, respectively.

By doing so, due to the difference in the wavelengths included in the illumination light, appropriate white-balance values for the return-light images differ from each other between when the filter is inserted at the post-stage of the white-light source and when the filter is removed therefrom. It is possible to switch to white-balance values required for the return-light images in the respective cases by means of the white-balance switching portion.

In the above-described invention, the light-source portion may intermittently irradiate the biological tissue with another type of excitation light that generates another type of fluorescence having a different wavelength from the illumination light and the fluorescence.

By doing so, it is possible to simultaneously capture the two types of fluorescences when the other type of excitation light is radiated onto the biological tissue.

In the above-described invention, the light-source portion may be provided with a near-infrared light source that emits the another type of excitation light in the form of near-infrared light.

By doing so, it is possible to simultaneously capture the fluorescence in the visible region and the fluorescence in the near-infrared region.

### {Advantageous Effects of Invention}

With the present invention, an advantage is afforded in that it is possible to concurrently acquire a white-light image and a visible-region fluorescence image without decreasing the frame rate of the white-light image.

### {Brief Description of Drawings}

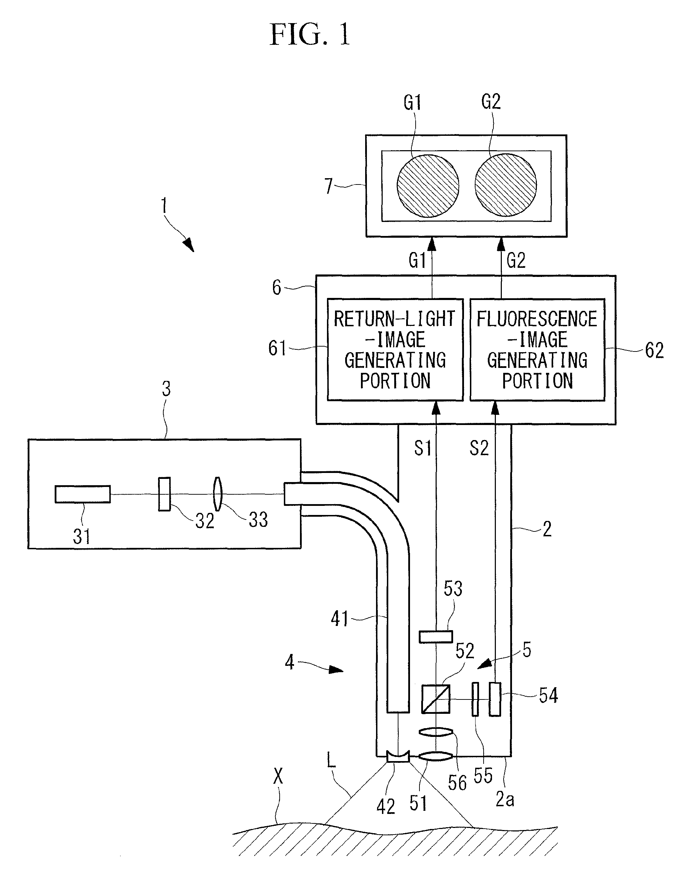

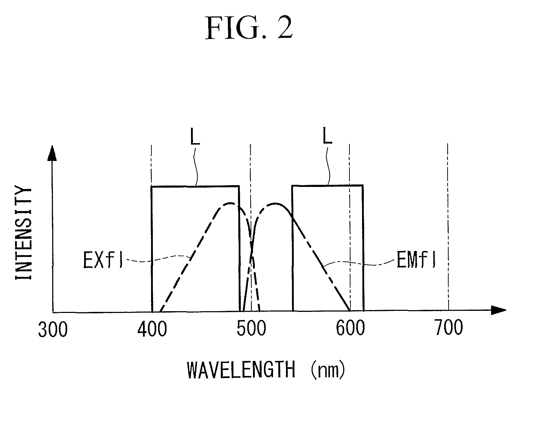

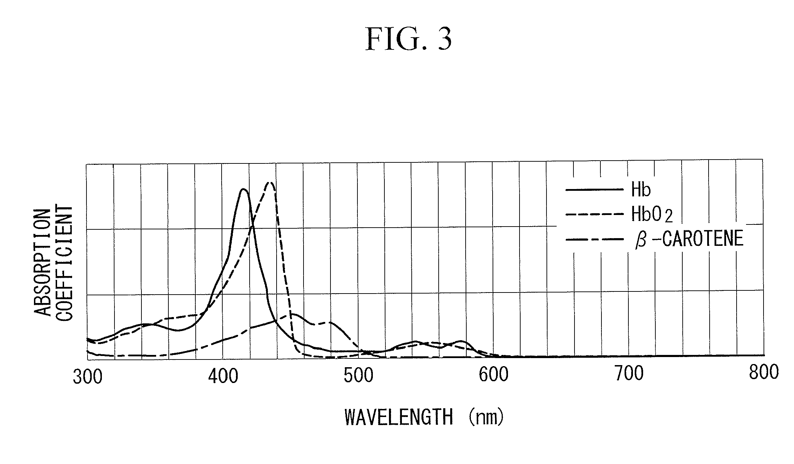

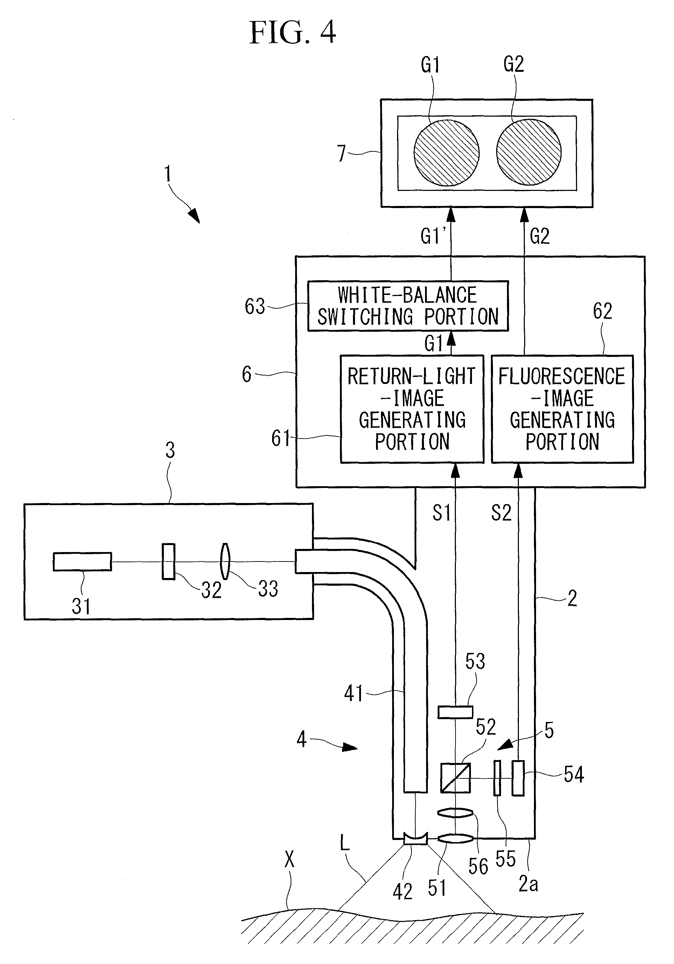

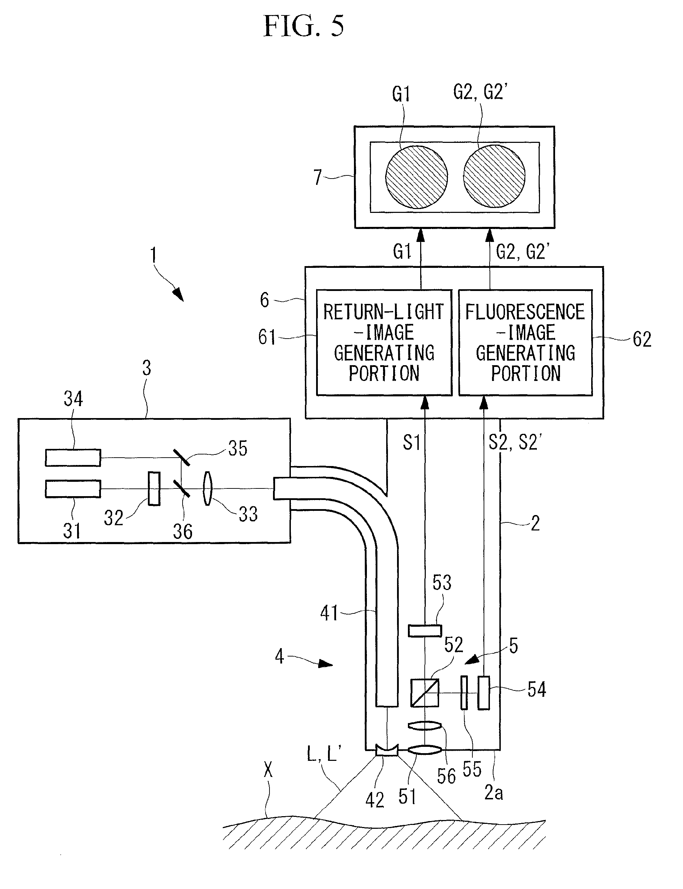

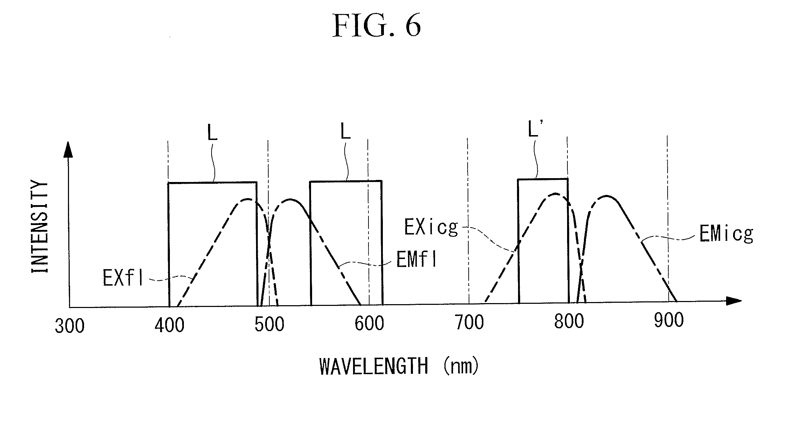

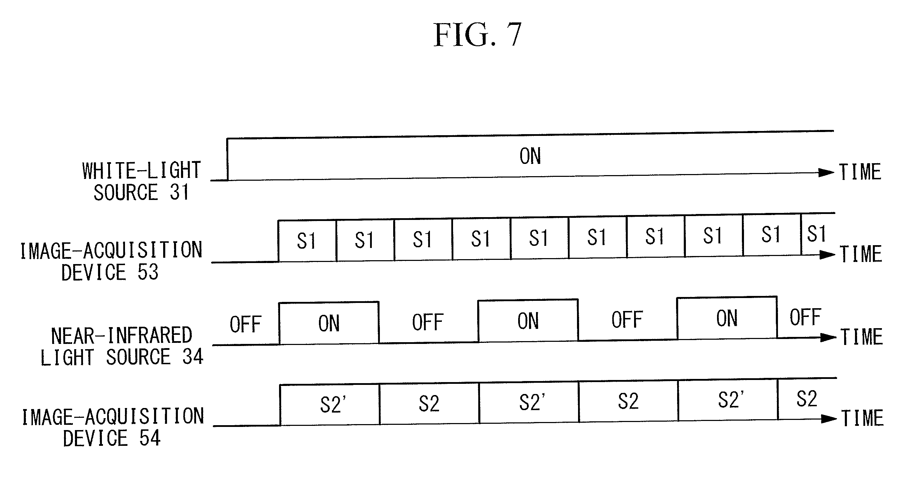

{Fig. 1} Fig. 1 is an overall configuration diagram showing a fluoroscopy apparatus according to a first embodiment of the present invention.
{Fig. 2} Fig. 2 shows spectra of illumination light output from a light-source unit in Fig. 1 and fluorescence excited by excitation light included in the illumination light.
{Fig. 3} Fig. 3 is a diagram for explaining the effect of the illumination light on biological tissue, and shows absorption spectra of major light absorbers existing in the biological tissue.
{Fig. 4} Fig. 4 is an overall configuration diagram showing a modification of the fluoroscopy apparatus in Fig. 1.
{Fig. 5} Fig. 5 is an overall configuration diagram showing a fluoroscopy apparatus according to a second embodiment of the present invention.
{Fig. 6} Fig. 6 shows spectra of illumination light and near-infrared light output from a light-source unit in Fig. 5 and two types of fluorescences excited by two types of excitation light included in the illumination light and the near-infrared light.
{Fig. 7} Fig. 7 is a timing chart for explaining the operation of the fluoroscopy apparatus in Fig. 5.

### {Description of Embodiments}

### {First Embodiment}

A fluoroscopy apparatus 1 according to a first embodiment of the present invention will be described below with reference to Figs. 1 to 4.

As shown in Fig. 1, the fluoroscopy apparatus 1 according to this embodiment is an endoscope apparatus provided with a long, thin inserted portion 2 that is inserted into a body, a light-source unit (light-source portion) 3, an illumination unit 4 that radiates illumination light L coming from the light-source unit 3 toward biological tissue X from a tip 2a of the inserted portion 2, an image-acquisition unit 5 that is provided at the tip 2a of the inserted portion 2 and that acquires image information S1 and S2 about the biological tissue X, an image-processing unit 6 that processes the image information S1 and S2 acquired by the image-acquisition unit 5, and a display portion 7 that displays images G1 and G2 processed by the image-processing unit 6.

The light-source unit 3 is provided with a white-light source 31 like a xenon lamp, a filter 32 that generates the illumination light L by extracting some wavelengths from white light emitted from the white-light source 31, and a coupling lens 33 that focuses the illumination light L generated by the filter 32. The white-light source 31 emits white light having wavelengths covering the entire visible region. The filter 32 allows light of at least wavelengths of 400 nm to 490 nm and of 540 nm to 610 nm to pass therethrough and blocks light of wavelengths of 490 nm to 540 nm. By doing so, in the visible region, a portion of the wavelength region is removed, and thus, substantially white light having a bipolar wavelength distribution, that is, the illumination light L, is generated, as indicated by solid lines in Fig. 2.

The illumination unit 4 is provided with a light-guide fiber 41 that is disposed in the inserted portion 2 in the longitudinal direction over nearly the entire length thereof and an illumination optical system 42 that is provided at the tip 2a of the inserted portion 2. The light-guide fiber 41 guides the illumination light L focused by the coupling lens 33. The illumination optical system 42 spreads out the illumination light L that has been guided thereto by the light-guide fiber 41 and radiates the illumination light L onto the biological tissue X which faces the tip 2a of the inserted portion 2.

The image-acquisition unit 5 is provided with an objective lens 51 that collects light from the biological tissue X, a beam splitter 52 that splits the light collected by the objective lens 51 into two beams, an image-acquisition device 53, such as a color CCD, and an image-acquisition device 54, such as a high-sensitivity monochromatic CCD, that respectively capture beams of light split by the beam splitter 52, and a barrier filter 55 disposed between the beam splitter 52 and the image-acquisition device 54.

The reference sign 56 indicates an imaging lens that forms images of the light collected by the objective lens 51 at image-acquisition surfaces of the respective image-acquisition devices 53 and 54.

Of the light incident thereon from the beam splitter 52, the barrier filter 55 blocks the return light of the illumination light L and selectively allows fluorescence, described below, to pass therethrough.

The image-processing unit 6 is provided with a return-light-image generating portion 61 that generates the return-light image G1 from the return-light-image information S1 acquired by the image-acquisition device 53 and a fluorescence-image generating portion 62 that generates the fluorescence image G2 from the fluorescence-image information S2 acquired by the image-acquisition device 54. The return-light-image generating portion 61 and the fluorescence-image generating portion 62 individually output the return-light image G1 and the fluorescence image G2 to the display portion 7. The image-processing unit 6 may output the return-light image G1 and the fluorescence image G2 to the display portion 7 after appropriately applying image processing such as noise removal to the individual images.

The display portion 7 displays the return-light image G1 and the fluorescence image G2 side-by-side.

Next, the operation of the thus-configured fluoroscopy apparatus 1 will be described.

In order to observe the biological tissue X by using the fluoroscopy apparatus 1 according to this embodiment, for example, a fluorescent dye that accumulates at a diseased portion is administered to the biological tissue X in advance. The fluorescent dye to be used is one that is excited by light of a wavelength between 450 nm and 490 nm and that generates fluorescence of a wavelength between 490 nm and 540 nm. In this embodiment, the fluorescent dye is assumed to be a fluorescein derivative that has an excitation wavelength EXfl between about 450 nm and 500 nm and a light-emission wavelength EMfl between about 510 nm and 540 nm, as shown in Fig. 2, and that is used as a cancer marker.

First, the inserted portion 2 is inserted into a body so that the tip 2a thereof is disposed facing the biological tissue X, and the illumination light L is radiated onto the biological tissue X from the tip 2a of the inserted portion 2 by activating the light-source unit 3. At the biological tissue X, the illumination light L is reflected at a surface of the biological tissue X, and a portion of the reflected illumination light L returns to the tip 2a of the inserted portion 2.

Here, the illumination light L includes excitation light of a wavelengths of 450 nm to 490 nm that excites the fluorescent dye. Therefore, the fluorescent dye contained in the biological tissue X is excited by the irradiation with the illumination light L, and a portion of the generated fluorescence returns to the tip 2a of the inserted portion 2 together with the return light of the illumination light L.

The return light of the illumination light L and the fluorescence that have been collected by the objective lens 51 at the tip 2a of the inserted portion 2 are split into two beams by the beam splitter 52. Then, one of the two beams is captured by the image-acquisition device 53 and is acquired as the return-light-image information S1, and the other one of the two beams is captured by the image-acquisition device 54 after only the fluorescence is extracted therefrom by the barrier filter 55, and is acquired as the fluorescence-image information S2. Here, although the image-acquisition device 53 captures both the return light of the illumination light L and the fluorescence, because the fluorescence is sufficiently weaker than the return light, the return-light-image information S1 acquired by the image-acquisition device 53 mainly contains morphological information of the biological tissue X.

Next, at the image-processing unit 6, the return-light image G1 is generated from the return-light-image information S1, and the fluorescence image G2 is generated from the fluorescence-image information S2. Then, the return-light image G1 and the fluorescence image G2 are displayed on the display portion 7.

Here, effects of the illumination light L on the biological tissue X will be described. Fig. 3 shows absorption spectra of major absorbers existing in the biological tissue X. As shown in Fig. 3, deoxyhemoglobin (Hb) and oxyhemoglobin (HbO₂), which are present in blood, strongly absorb light of wavelengths between 400 nm and 450 nm at a surface layer of the biological tissue X, and strongly absorb light of wavelengths between 540 nm and 565 nm at a deeper layer of the biological tissue X. β-carotene, which accumulates in adipose, strongly absorbs light of wavelengths between 450 nm and 490 nm. In addition, light of a wavelength between 600 nm and 610 nm is absorbed only slightly by all of Hb, HbO₂, and β-carotene.

These facts indicate that it is possible to capture the morphology of blood vessels existing in a surface layer and a deeper layer of the biological tissue X by using light of wavelengths of 400 nm to 450 nm and light of wavelengths of 540 nm to 565 nm as the illumination light L; it is possible to capture the morphology of adipose that is abundantly present at a surface of an organ or under a mucous membrane by using light of wavelengths of 450 nm to 490 nm as the illumination light L; and it is possible to capture the morphology of the surface of the biological tissue X by using light of wavelengths of 580 nm to 610 nm as the illumination light L.

In order to acquire morphological information of the biological tissue X, it is important to acquire information mainly about blood vessels, adipose, and surface shapes. With this embodiment, the illumination light L includes the wavelength regions in which adipose and blood vessels absorb light therein and the wavelength region in which none of them absorbs light therein. Therefore, as with a white-light image acquired by illuminating the biological tissue X with white light, it is possible to acquire the return-light image G1 in which the morphology of the biological tissue X is sufficiently clearly captured.

In addition, as described above, the wavelength region between 490 nm and 540 nm, which is not an important wavelength region for acquiring the morphological information of the biological tissue X and which carries a low amount of morphological information, is removed from the illumination light L, and the fluorescence is generated by using the illumination light L in the wavelength region between 490 nm and 540 nm, which is the wavelength region removed from the illumination light L; therefore, there is an advantage in that it is possible to concurrently acquire both the return-light image G1 and the fluorescence image G2 without decreasing the frame rate of the return-light image G1.

Note that, in this embodiment, the filter 32 may be provided in an optical path between the white-light source 31 and the coupling lens 33 in an insertable/removable manner.

By doing so, it is possible to simultaneously acquire both the return-light image G1 and the fluorescence image G2, as described above, by inserting the filter 32 into the optical path between the white-light source 31 and the coupling lens 33. On the other hand, when observing only the return-light image G1, by removing the filter 32 from the optical path, it is possible to irradiate the biological tissue X with the white light that has a wavelength covering the entire visible region as the illumination light L, and to acquire the return-light image G1 in which the color of the biological tissue X is more accurately produced.

As shown in Fig. 4, in the case in which the filter 32 is configured in an insertable/removable manner, it is preferable that the image-processing unit 6 be provided with a white-balance switching portion 63 that switches the white balance of the return-light image G1.

Due to the difference in the wavelengths included in the illumination light L, the appropriate white balance of the return-light image G1 acquired when the filter 32 is inserted into the optical path and that of the return-light image G1 acquired when the filter 32 is removed from the optical path differ from each other. Therefore, by switching the white balance, specifically, by switching a white-balance value of the return-light image G1 acquired when the filter 32 is inserted into the optical path and a white-balance value of the return-light image G1 acquired when the filter 32 is removed from the optical path to appropriate values, respectively, it is possible to always display the biological tissue X in the proper color on the display portion 7.

### {Second Embodiment}

Next, a fluoroscopy apparatus 1 according to a second embodiment of the present invention will be described with reference to Figs. 5 to 7. In this embodiment, configurations differing from those of the first embodiment will mainly be described, configurations common with those of the first embodiment will be given the same reference signs, and descriptions thereof will be omitted.

As shown in Fig. 5, the fluoroscopy apparatus 1 according to this embodiment mainly differs from that of the first embodiment in that the light-source unit 3 radiates another type of excitation light onto the biological tissue X and that two types of fluorescence images G2 and G2' are acquired.

Specifically, in addition to the above-described illumination light L that includes excitation light in the visible region, the light-source unit 3 outputs near-infrared light L' (for example, wavelengths of 750 nm to 800 nm) as the other type of excitation light. In Fig. 5, the light-source unit 3 is further provided with a near-infrared light source 34 for outputting the near-infrared light L', a mirror 35 and a dichroic mirror 36 that combine the near-infrared light L' from the near-infrared light source 34 with the light on the output optical axis of the white-light source 31. In accordance with control signals from a control portion (not shown), the light-source unit 3 intermittently outputs the near-infrared light L' from the near-infrared light source 34 in synchronization with the image-acquisition timing of the image-acquisition device 54.

Of the light incident thereon the beam splitter 52, the barrier filter 55 blocks the return light of the illumination light L and the near-infrared light L', and selectively allows the two types of fluorescences generated by the illumination light L and the near-infrared light L' to pass therethrough.

Next, the operation of the thus-configured fluoroscopy apparatus 1 will be described with reference to Figs. 6 and 7.

In order to observe the biological tissue X by using the fluoroscopy apparatus 1 according to this embodiment, for example, two types of fluorescent dyes that accumulate at diseased portions are administered to the biological tissue X in advance.

Here, as with the first embodiment, fluorescein is used as one of the fluorescent dyes. As the other fluorescent dye, one that is excited by the near-infrared light L' and that generates fluorescence in a wavelength region differing from those of the illumination light L and the fluorescence from fluorescein is used. In this embodiment, the other fluorescent dye is assumed to be indocyaningreen (ICG) that has an excitation wavelength EXicg, which peaks at about 780 nm, and a light-emission wavelength EMicg, which peaks at about 845 nm, as shown in Fig. 6, and that is used to obtain blood vessels.

As with the first embodiment, the illumination light L is radiated onto the biological tissue X from the tip 2a of the inserted portion 2. At this time, as shown in Fig. 7, the near-infrared light L' from the light-source unit 3 is repeatedly and alternately output and stopped each time the image-acquisition device 54 acquires one-frame worth of the fluorescence-image information S2 and S2'. By doing so, when outputting of the near-infrared light L' is stopped, as with the first embodiment, the image-acquisition device 54 acquires the fluorescence-image information S2 for which the fluorescence from only fluorescein is captured. On the other hand, when the near-infrared light L' is being output, the image-acquisition device 54 acquires the fluorescence-image information S2' for which fluorescences from both fluorescein and ICG are captured. The fluorescence-image information S2 and S2' are alternately input to the fluorescence-image generating portion 62.

At the fluorescence-image generating portion 62, fluorescence images G2, in which fluorescence from fluorescein is captured, and fluorescence images G2', in which fluorescences from both fluorescein and ICG are captured, are alternately generated from the alternately-input fluorescence-image information S2 and S2'. Note that the image-processing unit 6 may generate a fluorescence image in which the fluorescence from only ICG is captured by subtracting, from a fluorescence image G2', a fluorescence image G2 that is generated immediately before that fluorescence image G2', and this fluorescence image may be output to the display portion 7.

As above, with this embodiment, in addition to the advantage of the first embodiment, there is a further advantage in that it is possible to acquire two types of fluorescence images G2 and G2' without decreasing the frame rate of the return-light image G1 by generating another type of fluorescence in the near-infrared region that does not interfere with the illumination light L and fluorescence in the visible region.

### {Reference Signs List}

1 fluoroscopy apparatus
2 inserted portion
3 light-source unit (light-source portion)
31 white-light source
32 filter
33 coupling lens
34 near-infrared light source
35 mirror
36 dichroic mirror
4 illumination unit
41 light-guide fiber
42 illumination optical system
5 image-acquisition unit
51 objective lens
52 beam splitter
53, 54 image-acquisition device
55 barrier filter
56 imaging lens
6 image-processing unit
61 return-light-image generating portion
62 fluorescence-image generating portion
63 white-balance switching portion
7 display portion
L illumination light
L' near-infrared light
G1 return-light image
G2, G2' fluorescence image

## Claims

1. A fluoroscopy apparatus comprising:
a light-source portion that irradiates biological tissue with illumination light including excitation light;
a return-light-image generating portion that generates a return-light image of the biological tissue by capturing return light returning from the biological tissue irradiated with the illumination light from the light-source portion; and
a fluorescence-image generating portion that generates a fluorescence image by capturing fluorescence generated at the biological tissue due to irradiation thereof with the excitation light from the light-source portion,
wherein the illumination light is visible light that does not include at least a portion of the wavelength region between 490 nm and 540 nm, and
the excitation light includes some wavelengths of the illumination light and generates fluorescence having a wavelength included in the at least a portion of the wavelength region.

2. The fluoroscopy apparatus according to Claim 1, wherein the at least a portion of the wavelength region is 510 nm to 530 nm.

3. The fluoroscopy apparatus according to Claim 1 or 2, wherein the at least a portion of the wavelength region has a width equal to or greater than 20 nm.

4. The fluoroscopy apparatus according to Claim 1, wherein the light-source portion is provided with
a white-light source that emits white light and
a filter that removes light of the at least a portion of the wavelength region between 490 nm and 540 nm from the white light emitted from the white-light source,
wherein the filter is provided at a post-stage of the white-light source in an insertable/removable manner, and
the illumination light is substantially white light that has passed through the filter.

5. The fluoroscopy apparatus according to Claim 4, further comprising:
a white-balance switching portion that switches white balances of the return-light images generated by the return-light-image generating portion, when the filter is inserted at the post-stage of the white-light source and when the filter is removed from the post-stage of the white-light source, respectively.

6. The fluoroscopy apparatus according to any one of Claims 1 to 5, wherein the light-source portion intermittently irradiates the biological tissue with another type of excitation light that generates another type of fluorescence having a different wavelength from the illumination light and the fluorescence.

7. The fluoroscopy apparatus according to Claim 6, wherein the light-source portion is provided with a near-infrared light source that emits the another type of excitation light in the form of near-infrared light.
